# EUROPÄISCHE PATENTANMELDUNG

(11) **EP 2 567 703 A2**
(43) Veröffentlichungstag der Anmeldung: **13.03.2013**
(21) Anmeldenummer: 12195762.5
(22) Anmeldetag: 23.12.2008
(51) Int. Cl.: A61K 36/28, A61P 1/16

(54) **Neuer Mariendistelextrakt, Verfahren zur Herstellung und Verwendung**

(30) Priorität: 23.12.2007 DE 102007063115; 23.08.2008 DE 102008039271
(62) Teilanmeldung aus: 08865199.7
(71) Anmelder: Euromed SA, 08100 Mollet del Vallès (ES)
(72) Erfinder: Nagell, Astrid, 22399 Hamburg (DE); Aguirre, Jaime Xiol, 08011 Barcelona (ES); Prous, Santiago Rull, 08034 Barcelona (ES)
(74) Vertreter: Simandi, Claus

(57) **Zusammenfassung**

Die vorliegende Erfindung betrifft ein Verfahren zur Herstellung eines Mariendistelfrüchte-Extrakt, insbesondere eine Flavanolignan-Zubereitung mit erhöhter Freisetzungsrate und verbesserter Resorbierbarkeit und dessen Verwendung, insbesondere zur Therapie und Prophylaxe von Lebererkrankungen.

## Beschreibung

Die vorliegende Erfindung betrifft ein Verfahren zur Herstellung eines Mariendistelfrüchte-Extrakt, insbesondere eine Flavanolignan-Zubereitung mit erhöhter Freisetzungsrate und verbesserter Resorbierbarkeit und dessen Verwendung, insbesondere zur Therapie und Prophylaxe von Lebererkrankungen.

Die Mariendistel (Milk thistle, Silybum marianum oder Carduus marianus) ist eine besonders in Südwest- und Mitteleuropa (Österreich, Ungarn) kultivierte Pflanze und ist in Eurasien, Nordamerika und Südamerika neben Australien naturalisiert. Weitere Anbaugebiete befinden sich in China.

Die Wirksamkeit der Droge Mariendistel (Samen und Früchte) bei der Prophylaxe und Behandlung von verschiedenen Formen der Leber- und Gallendysfunktionen ist bekannt. Die Droge besteht aus den reifen, vom Pappus befreiten Früchten mit einem Mindestgehalt an Sylimarin von 1,5 % (Pharmacopoea Europaea (Ph. Eur.), europäisches Arzneibuch 2007). Bereits aus dem Altertum sind Tinkturen (zumeist alkoholische Auszüge aus der Droge) aus Mariendistel bekannt. Insbesondere isoliertes Silymarin ist besonders geeignet (z.B. DE 1 923 982, DE 1 767 666 (Madaus)).

Silymarin ist ein Flavanolignan-Komplex bzw. Polyhydroxyphenylchromanonen und wurde erstmals aus der Pflanze in den 1960 -iger Jahren isoliert (Dissertation Janiak Bernhard, Juni 1960, FU-Berlin (DE 2020407), Pelter A., Hänsel R., Tetrahedron Letters, 25, (1968)).

Silymarin besteht aus einem Gemisch mit dem Flavonolignan-Komplex I-IV und zwar in den Hauptbestandteilen aus den vier Flavanolignanen, Silybin (Silybinin) (Silymarin I), Silydianin (Silymarin II) und Silychristin (Silymarin III) 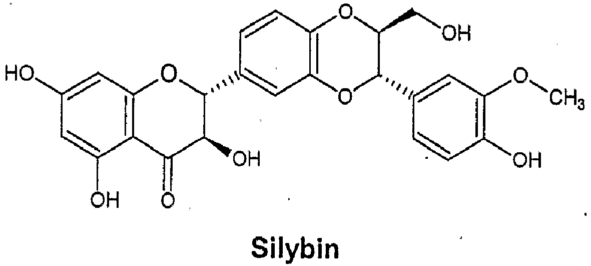 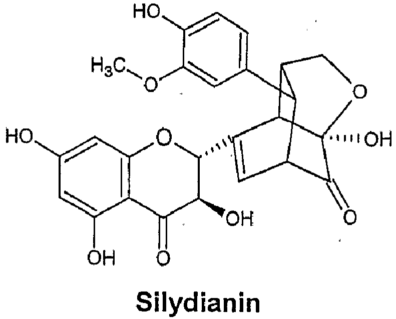 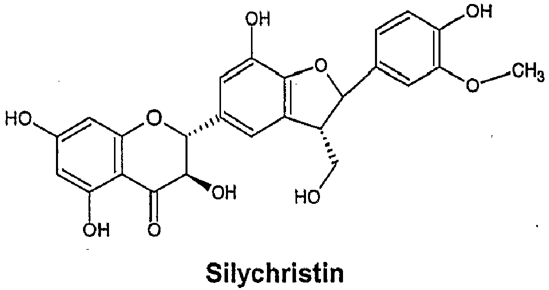 und Isosilibin (Silymarin IV). Bei diesen Flavolignanen ist das Taxifolin mit Coniferyl-Alkohol verknüpft.

Weitere bekannte Nebenbestandteile sind Dehydrosilybin, 3-Desoxysilychristin, Desoxysilydianin (Silymonin), Silyadrin, Silybinom, Silyermin und Neosilymerin.

Bevorzugt werden die Früchte der Mariendistel zur Extraktherstellung verwendet. Im Stand der Technik sind bereits solche Extrakte aus Mariendistel und Verfahren zu dessen Herstellung beschrieben, wie z.B. in DE 1 923 982, DE 29 14 330 (Madaus) offenbart.

Bekannt ist zudem ein Mariendistelfrüchtetrockenextrakt (Extr. Cardui mariae fruct. Siccum), welcher u.a. mit dem Extraktionsmittel Ethylacetat aus der Pflanzendroge erhalten wird und nach der geltenden Ph. Eur. standardisiert ist.

Die deklarierten Anforderungen an einen Trockenextrakt belaufen sich auf einen Gehalt von vorzugsweise 30 - 65 Gew. % Silymarin (andere Gehaltsspannen sind möglich), wobei der Anteil Silymarin folgende Anteile enthält:
40-65 Gew.%:
   Silibin(in) A und B (Diastereomerengemisch, C₂₅H₂₂OH₁₀ MG 482,4) und
10-20 Gew.%
   Isosilibinin A und B (Diastereomerengemisch , C₂₅H₂₂OH₁₀ MG 482,4) und
20-45 Gew.%:
   Silidanin und Silicristin (C₂₅H₂₂OH₁₀ MG 482, 4).

Zur Herstellung eines Extraktes wird das Rohmaterial (hier: Pflanzendroge) üblicher Weise entfettet, extrahiert, filtriert, (auf)konzentriert und gereinigt.

Üblicher Weise wird nach besagter kontinuierlicher Extraktion mit Ethylacetat / Ethanol / Aceton / Methanol (ggfs. wässrig) oder wässrigen Mischungen mit den vorher genannten Lösemitteln eine Filtration durchgeführt mit anschließender (Auf)Konzentration. Nachfolgend erfolgt eine Aufreinigung mit Ethanol und Hexan (weitere Entfettung), so dass der oben genannte Gehalt an Silymarin erhalten werden kann.

Eine solche Zusammensetzung erlaubt eine Freisetzungsrate an Silymarin von 30 - ca. 40 % (gemessen nach Ph. Eur. 5.7; 2.9.3 (01/2006:20903 in der jeweils gültigen Fassung, z.B. mittels Körbchenmethode, Paddle-Modell).

Es besteht jedoch ein hohes Bedürfnis die Freisetzungsrate von Silymarin im nativen Extrakt zu erhöhen.

Es ist bekannt, daß diese Flavano-Lignane in Wasser unlöslich bis schwer löslich sind (Löslichkeit von reinem Silymarin beträgt bei pH 6,9 ca. 0,08 mg/ml). Aufgrund dieses Lösungsverhaltens ist die Freisetzungsrate dieser Verbindungen und de facto ihre Bioverfügbarkeit / Resorbierbarkeit im Körper des Menschen oder Säugetier nicht ausreichend.

Zur Erhöhung der Freisetzungsrate wurde versucht die Flavano-Lignane zu derivatisieren, wie z.B. mittels Polyalkoholen, Aminozuckern, Estern oder mittels Einschlußverbindungen zu komplexieren, wie Cyclodextrin (EP 0 422 497 B1 (Madaus)), oder Komplexverbindungen, wie z.B. mittels Phosphatidylcholin.

Nachteilig ist jedoch, dass physiologische Fremdsubstanzen mit unerwünschten Nebenwirkungen entstehen können.

Im Stand der Technik ist ferner bekannt, dass die Freisetzungsrate durch Trägerstoffe wie z.B. 1-Vinyl-2-Pyrrolidon, Mannitol, u. a. erhöht werden kann (EP 0722 918 B1, US 5,906,991 (Madaus)). Ferner sind Netzmittel wie Polysorbate (Tensid) vonnöten. In EP 1 021 198 B1 (Madaus) wird ein Silymarin-Co-Präzipitat mithilfe von PEG vorgeschlagen. Diese genannten Verfahren haben jedoch alle den Nachteil, dass eine Dosierung erschwert wird und Fremdstoffe mit nicht genau definierten Nebenwirkungen entstehen können.

Daher besteht die Aufgabe einen verbesserten Mariendistelfrüchte-Extrakt bereit zu stellen, insbesondere einen solchen, der eine vorteilhaft erhöhte Silymarin-Freisetzungsrate unter Beibehaltung des nativen Charakters aufweist. Hierbei soll der Extrakt weitgehend ohne Zusatzstoffe, Bei- Zuschläge, Trägerstoffe, Netzmittel herzustellen sein.

Die Aufgabe wird dadurch gelöst, dass ein Verfahren zur Herstellung eines Mariendistelfrüchte-Extraktes bereit gestellt wird, wobei in den folgenden Schritten
a.) die Pflanzendroge mit einem Lösungsmittel mittlerer Polarität extrahiert wird (solche wie Ethylacetat, Ethanol, Aceton, Methanol, ggfs. wässrige Anteile enthaltend), vorzugsweise bei 40 - 80 Grad Celsius, besonders bevorzugt 50 - 70 Grad Celsius,
b.) getrennt, vorzugsweise filtriert,
c.) konzentriert, vorzugsweise im Vakuum unter Rühren bei einer Temperatur unter 60 Grad Celsius, vorzugsweise unter 40 Grad Celsius und c') ggfs. mit heißem Wasser gewaschen wird,
d.) in Ethanol, vorzugsweise 96 % Ethanol oder mehr oder einem Lösungsmittel ähnlicher Polarität, und anschließend in Hexan oder einem Lösungsmittel ähnlicher Polarität versetzt und eingeengt wird, vorzugsweise bei einem Druck von 1 - 100 mbar, und die erhaltene Ethanol-Wasser Phase abgetrennt wird,
e.) getrocknet und ggfs. zerkleinert wird
f.) in wasserfreiem Alkohol, vorzugsweise Ethanol aufgenommen wird,
g.) ggfs. filtriert und konzentriert wird und
h.) getrocknet und ggfs. zerkleinert wird.

Überraschender Weise führt der zusätzliche Schritt f.) zu einer signifikanten Erhöhung der Silymarin-Freisetzungsrate auf 80 % (siehe Vergleichsbeispiele). Dies ist besonders vorteilhaft, da eine geringere Dosierung des erfindungsgemäßen Mariendistelfrüchte-Extrakt erzielt wird. Ferner ist vorteilhaft, dass eine Qualität erreicht wird, wie sie im Stand der Technik nur mittels Zusatzstoffen, Bei- Zuschläge, Trägerstoffe und Netzmittel erreicht wird.

Der Begriff "wasserfreier Alkohol" im Schritt f.) umfasst vorzugsweise C1-C4 Alkohole, besonders bevorzugt Ethanol, wie 99%, gar 99,5 % rein.

Daher betrifft die Erfindung ebenfalls ein Verfahren zum Herstellen eines Mariendistelfrüchte-Extrakt mit einer Silymarin-Freisetzungsrate von 80 % und mehr, wobei ein Extrakt mit einem Gehalt von 15 - 85 Gew. %, insbesondere 30 - 65 Gew. % Silymarin mit wasserfreiem Alkohol aufgenommen wird, ggfs. filtriert und konzentriert wird und anschließend getrocknet und ggfs. zerkleinert wird.

Im Rahmen der vorliegenden Erfindung wird unter "Silymarin" ein Stoffgemisch bezeichnet, welches (mindestens) die vier Substanzen Silybin, Silydianin, Silychristin und Isosilbin in unterschiedlichen Konzentrationen enthalten. Dabei ist es unwesentlich, in welchen Verhältnissen diese Stoffe zueinander vorliegen und ob noch weitere Substanzen in dem Gemisch vorhanden sind. Bevorzugt ist jedoch, dass diese Stoffe die Anforderungen des Ph. Eur. oder DAB, in der jeweils geltenden Fassung erfüllen. Dies ist erfindungsgemäß der Fall.

Eine "Silymarin-Freisetzungsrate von 80 % und mehr" bedeutet, dass die Wirkstoffe zu mindestens 80 % in wässriger Lösung löslich sind (Standard gemäß Ph. Eur., siehe Beispiele).

Dies führt vorteilhaft zu einer verbesserten Resorbierbarkeit.

Die besondere Eignung des erhaltenen Mariendistelfrüchte-Extrakt ist darin zu sehen, dass durch den Verfahrensschritt f.) die kristallinen Anteile im erhaltenen Extrakt wesentlich reduziert sind und ein Mariendistelfrüchte-Extrakt mit einer im Wesentlichen amorphen Kristallmodifikation erhalten wird.

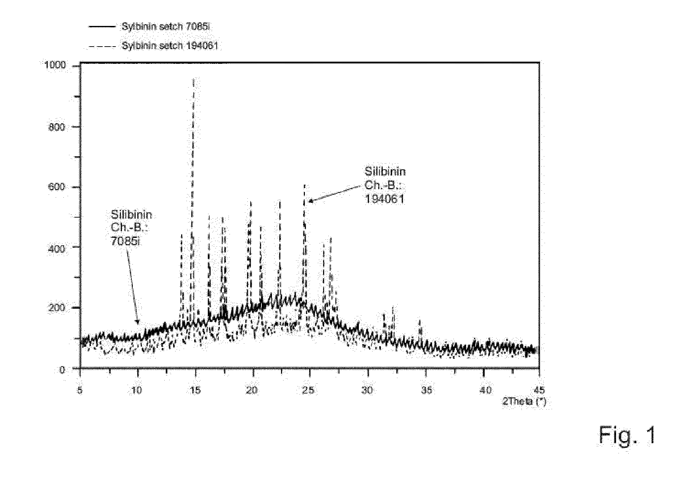

Daher betrifft die Erfindung einen neuen Mariendistelfrüchte-Extrakt oder Flavanolignan-Zubereitung, der im Wesentlichen eine amorphe Kristallmodifikation aufweist (siehe Vergleichsversuche der Röntgenstrukturanalyse in Figur 1).

In einer besonders bevorzugten Ausführungsform betrifft die Erfindung einen neuen Mariendistelfrüchte-Extrakt oder Flavanolignan-Zubereitung bestehend aus einer amorphen Kristallmodifikation, wobei der kristalline Anteil weniger als 20 %, vorzugsweise weniger als 10 %, besonders bevorzugt weniger als 7 %, gar 5 % beträgt.

Ferner betrifft die Erfindung daher ein Arzneimittel bestehend aus einem erfindungsgemäßen Mariendistelfrüchte-Extrakt oder dessen Verwendung zur Prophylaxe und Behandlung von Leber- und Gallendysfunktionen, insbesondere von toxischen Leberschäden (Fettleber, Alkohol), Hepatosen wie Pilzvergiftungen, akutes Leberversagen, Lebernekrose, Leberdystrophie, Leberzirrhose, Leberfibrose, Lebervergrößerung und Leberverfettung, Leberinsuffizienz, Hepatitis, insbesondere Hepatitis C.

Ferner betrifft die Erfindung eine pharmazeutische Formulierung enthaltend ein erfindungsgemäßes Arzneimittel bestehend aus einem erfindungsgemäßen Mariendistelfrüchte-Extrakt.

Die erfindungsgemäßen Mariendistelfrüchte-Extrakte können in Form von pharmazeutischen Zubereitungen in Dosierungseinheiten zubereitet werden. Dies bedeutet, dass die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben, einem Drittel oder einem Viertel einer Tagesdosis entspricht. Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Formulierungen seien Tabletten, Dragees, Kapseln, Pillen, Granulate, Suppositorien, Lösungen, Saft, Suspensionen und Emulsionen, genannt. Tabletten, Dragees, Kapseln, Pillen und Granulate können den oder die Wirkstoffe neben den üblichen Trägerstoffen enthalten, wie a) Füll- und Streckmittel, z.B. Stärken, Milchzucker, Rohrzucker, Glukose, Mannit und Kieselsäure, b) Bindemittel, z.B. Carboxymethylcellulose, Alginate, Gelatine, Polyvinylpyrrolidon, c) Feuchthaltemittel, z.B. Glycerin, d) Sprengmittel, z.B. Agar-Agar, Calciumcarbonat und Natriumcarbonat, e) Lösungsverzögerer, z.B. Paraffin und f) Resorptionsbeschleuniger, z.B. quartäre Ammoniumverbindungen, g) Netzmittel, z.B. Cetylalkohol, Glycerinmonostearat, h) Adsorptionsmittel, z.B. Kaolin und Bentonit und i) Gleitmittel, z.B. Talkum, Calcium- und Magnesiumstearat und feste Polyethylenglykole oder Gemische der unter a) bis i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. C14-Alkohol mit C16-Fettsäure) oder Gemische dieser Stoffe.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3- Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten. Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

### Beispiele und Figuren:

Diese Beispiele dienen ausschließlich zur Erläuterung der Erfindung, ohne die Erfindung auf diese Beispiele zu begrenzen.

### Beispiel 1:

### Vergleichsversuche zur Freisetzung von Silymarin:

Es wurden vergleichende Extrakte (Silibinin Ch.-B.: 194051, Ch.-B.: 7085i) gemäß obiger Beschreibung hergestellt, wobei Silibinin Ch.-B.: 7085i mittels dem zusätzlichen Verfahrensschritt f.) hergestellt wurde.

Bei pH 7.5 unter den nach Ph. Eur. angegebenen Bedingungen (Dissolution test of solids; Ph. Eur 5.7; 2.9.3 (01/2006:20903) ergibt sich eine (Silymarin) Wirkstoff-Freisetzung von:

| Probe | Probenahme | Gelöste Menge in % |
|---|---|---|
| Silibinin Ch.-B.: 194051 | Nach 30 Min. | 4.16% |
| Silibinin Ch.-B.: 7085i | Nach 30 Min. | 49.13% |

Die Ergebnisse zeigen, daß durch die Behandlung mit wasserfreiem Ethanol im Schritt f.) das vorher schwer lösliche Silibinin-Gemisch, bestehend aus amorphen und kristalliner Struktur, in eine amorphe Kristallmodifikation (siehe Figur 1) überführt wird (d.h. die Kristallgitterstruktur ist verändert), die eine bessere Löslichkeit bzw. Wirkstofffreisetzung zur Folge hat.

Durch diesen zusätzlichen Verfahrensschritt ist es gelungen, den oben bezeichneten Extrakt mit einer Wirkstoff-Freisetzung von mindestens 80% Gesamtsilymarin, berechnet als Silibinin (HPLC- Ph. Eur.01/2007:2071) nach 30 min. herzustellen, da durch dieses Verfahren nicht nur das Silibinin sondern auch die anderen Silymarin-Isomeren in ihrer Löslichkeit verbessert wurden.

Figur 1 zeigt die veränderte Kristallmodifikation aus dem Vergleich von Silibinin Ch.-B.: 194051 und Silibinin Ch.-B.: 7085i mittels Röntgenstrukturanalyse (Bedingungen gemäß Beispiel 2).

Röntgenographische Untersuchungen wurden an einem Diffraktometer X'Pert Pro MPD der Firma PANalytical B.V. mit Bragg-Brentano Geometrie und X'Celerator Detektor durchgeführt.

### Weitere vergleichende Versuchsdurchführungen:

### Beispiel 2:

### Verwendete Methodik

### a.) Probenvorbereitung:

### Zwei Feststaubproben von Produkten

Ref. 7233i mit Schritt f.) gemäß dem erfindungsgemäßen Verfahren und Ref. 7232i ohne Schritt f.)

### b.) Röntgenbeugungsanalyse mit der Staubmethode

Einführung von Teilen des Staubmaterials, das in Lindemann-Glaskapillaren mit 0,5 Millimeter Durchmesser zugeführt wird.

### c.) Ausstattung und Versuchsbedingungen:

Beugungsmesser PANalytical X'Pert PRO MPD mit Winkelmesser 9/9 mit einem Radius von 240 Millimetern, Paralleloptik mit Hybridmonochromator und Übertragungsgeometrie mit Probenträgern für Kapillaren mit Spinner.

Cu-Ka-Strahlung (k = 1.5406 Å).

Arbeitsleistung: 45 kV - 40 mA.

Slit, der die Menge mit 0,19 Millimeter festlegt

Soller-Fenster mit 0,02 Strahlung in der einfallenden Menge und in der gebeugten Menge

### d.) Messgerät X'Celerator mit einer aktiven Länge von 2122.

Spülungen 29 von 3 bis 60° 29 mit einer Schrittgröße von 0,017 und einer Messzeit von 1500 Sek. pro Schritt.

### e.) Ziel

Erzeugung der Diagramme der Röntgenbeugung mit der Staubmethode. Feststellung der Kristallisationsgrade.

### f.) Methodik

Der Kristallisationsgrad ist der Prozentsatz im Gewicht der kristallinen Phase bei einem Probengemisch aus kristallinen und amorphen Phasen, und zwar mit einem Kristallisationsindex, Ci:
Ci = 100[Xc/(Xa+Xc)] wobei Xc die Fraktion im Gewicht der amorphen Phasen darstellt.

Die Werte von Xc wurden aus der Summe der Bereiche aller engen Punkte (der kristallinen Phase) im Winkelbereich der Studie ermittelt. Die Werte von Xa wurden durch die Bestimmung der Bereiche der breiten Punkte oder 'Halos' (der amorphen Phase) ermittelt.

### g.) Ergebnisse

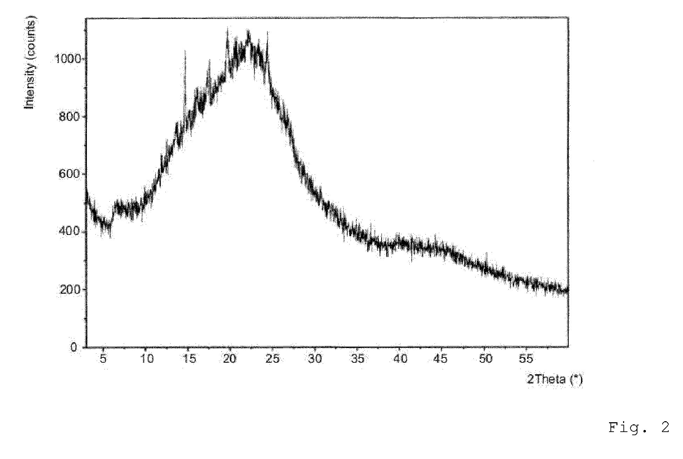

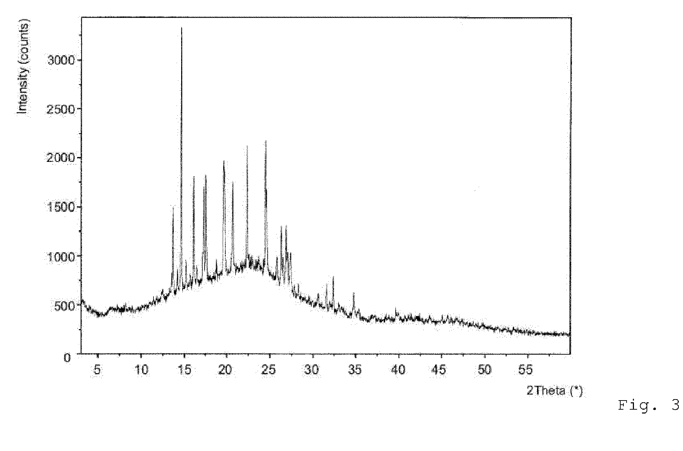

Die Figuren 2 und 3 stellen die erhaltenen Diagramme im kompletten Winkelmessbereich dar. Es handelt sich um Probengemische aus kristallinen und amorphen Phasen.

Die erhaltenen Ci-Werte lagen im Fall der Probe Ref. 7233i bei 7% (siehe Figur 2)und im Fall der Probe Ref. 7232i bei 24% (siehe Figur 3).

## Patentansprüche

1. Verfahren zum Herstellen eines Mariendistelfrüchte-Extrakt mit einer Silymarin-Freisetzungsrate von 80 % und mehr, **dadurch gekennzeichnet, dass** ein Extrakt enthaltend 15 -85 Gew. %, insbesondere 30 - 65 Gew. % Silymarin mit wasserfreiem Alkohol aufgenommen wird, ggfs. filtriert und konzentriert wird und anschließend getrocknet und ggfs. zerkleinert wird.

2. Verfahren zum Herstellen eines Mariendistelfrüchte-Extrakt mit einer Silymarin-Freisetzungsrate von 80 % und mehr, **dadurch gekennzeichnet, dass**
a.) die Pflanzendroge mit einem Lösungsmittel mittlerer Polarität extrahiert wird, vorzugsweise bei 40 - 80 Grad Celsius, besonders bevorzugt 50 - 70 Grad Celsius,
b.) getrennt, vorzugsweise filtriert,
c.) konzentriert, vorzugsweise im Vakuum unter Rühren bei einer Temperatur unter 60 Grad Celsius, vorzugsweise unter 40 Grad Celsius und c') ggfs. mit heißen Wasser gewaschen wird,
d.) in Ethanol, vorzugsweise 96 % Ethanol oder mehr oder einem Lösungsmittel ähnlicher Polarität, und anschließend in Hexan oder einem Lösungsmittel ähnlicher Polarität versetzt und eingeengt wird, vorzugsweise bei einem Druck von 1 - 100 mbar, und die erhaltene Ethanol-Wasser Phase abgetrennt wird,
e.) getrocknet und ggfs. zerkleinert wird
f.) in wasserfreiem Alkohol, vorzugsweise Ethanol aufgenommen wird,
g.) ggfs. filtriert und konzentriert wird und
h.) getrocknet und ggfs. zerkleinert wird.

3. Verfahren zum Herstellen eines Mariendistelfrüchte-Extrakt mit einer Silymarin-Freisetzungsrate von 80 % und mehr nach Anspruch 2, **dadurch gekennzeichnet, dass** im Schritt a.) das Lösungsmittel mittlerer Polarität ausgewählt ist aus der Gruppe Ethylacetat, Ethanol, Methanol, vorzugsweise Ethylacetat.

4. Verfahren zum Herstellen eines Mariendistelfrüchte-Extrakt mit einer Silymarin-Freisetzungsrate von 80 % und mehr nach Anspruch 2, **dadurch gekennzeichnet, dass** im Schritt f.) der wasserfreie Alkohol ein wasserfreier C1-C4 Alkohol ist.

5. Mariendistelfrüchte-Extrakt erhältlich nach einem Verfahren gemäß einem der Ansprüche 1 bis 4.

6. Mariendistelfrüchte-Extrakt bestehend im Wesentlichen aus einer amorphen Kristallmodifikation.

7. Mariendistelfrüchte-Extrakt bestehend aus einer amorphen Kristallmodifikation, wobei der kristalline Anteil weniger als 20 %, insbesondere weniger als 10 %, vorzugsweise weniger als 7 % beträgt.

8. Mariendistelfrüchte-Extrakt nach einem der Ansprüche 5 bis 7 zur Prophylaxe und Behandlung von Leber- und Gallendysfunktionen, insbesondere von toxischen Leberschäden (Fettleber, Alkohol), Hepatosen wie Pilzvergiftungen, akutes Leberversagen, Lebernekrose, Leberdystrophie, Leberzirrhose, Leberfibrose, Lebervergrößerung und Leberverfettung, Leberinsuffizienz, Hepatitis, insbesondere Hepatitis C.

9. Pharmazeutische Zubereitung enthaltend ein Mariendistelfrüchte-Extrakt nach Anspruch 8.
